# DEMANDE DE BREVET EUROPEEN

(11) **EP 1 647 264 A1**
(43) Date de publication de la demande: **19.04.2006**
(21) Numéro de dépôt: 05292144.2
(22) Date de dépôt: 13.10.2005
(51) Int. Cl.: A61K 8/40, A61Q 5/06

(54) **Utilisation pour le traitement cosmétique des matières kératiniques, d'une composition filmogène comprenant un monomère électrophile et un polymère non siliconé, conférant un gainage rigide.**

(30) Priorité: 13.10.2004 FR 0410805
(71) Demandeur: L'OREAL, 75008 Paris (FR)
(72) Inventeur: Vic, Gabin, 60280 Venette (FR); Brun, Gaelle, 75015 Paris (FR); Livoreil, Aude, 75006 Paris (FR); Gourlaouen, Luc, 92600 Asnieres (FR); Giroud, Franck, 92110 Clichy (FR); Rollat-Corvol, Isabelle, 75017 Paris (FR)
(74) Mandataire: Casalonga, Axel

(57) **Abrégé**

La présente invention concerne l'utilisation pour le traitement cosmétique des matières kératiniques, d'une composition filmogène comprenant dans un milieu cosmétiquement acceptable, au moins un monomère électrophile et au moins un polymère non siliconé tel que le film obtenu à partir de la composition par séchage à température ambiante et avec un taux d'humidité relative d'environ 50 % présente un module d'Young compris entre 100 et 2000 MPa, mesuré pour une épaisseur de 0,5 mm et avec une vitesse de traction de 20 mm/min.

## Description

La présente invention est relative à une utilisation pour le traitement cosmétique des matières kératiniques d'une composition filmogène comprenant au moins un monomère électrophile et au moins un polymère non siliconé particulier, et à un procédé de traitement cosmétique mettant en oeuvre une telle composition.

Dans le domaine de la cosmétique, on cherche à modifier les propriétés superficielles des matières kératiniques telles que les fibres kératiniques comme les cheveux, par exemple pour apporter aux cheveux un effet conditionnant comme la douceur, ou de la brillance. Pour ce faire, on utilise généralement des compositions cosmétiques à base d'agents de conditionnement tels que des silicones ou des polymères ayant une forte affinité pour les matières kératiniques, et notamment pour les cheveux.

Cependant, le gainage des fibres kératiniques telles que les cheveux obtenu avec ces compositions présentent souvent un toucher collant désagréable ainsi que l'inconvénient d'un transfert par exemple, lorsqu'une personne passe la main dans les cheveux. Ce phénomène de transfert laisse une impression de cheveux sales ou collants.

En outre, ces agents de conditionnement ont tendance à s'éliminer au cours de lavage avec des shampoings, rendant nécessaire le renouvellement des applications des compositions sur les cheveux.

Pour accroître la rémanence de dépôt de polymères, il peut être envisagé d'effectuer une polymérisation radicalaire de certains monomères directement sur les cheveux. Toutefois, on constate une forte dégradation des fibres capillaires liées probablement aux amorceurs de polymérisation et les cheveux ainsi traités sont difficilement démêlables.

La Demanderesse a trouvé de manière surprenante qu'en utilisant l'association d'au moins un monomère électrophile tel que décrit ci-dessous à au moins un polymère non siliconé particulier, il était possible de surmonter les inconvénients des gainages de l'art antérieur et d'obtenir un conditionnement et une brillance améliorés des cheveux, et ce de manière durable.

Le polymère non siliconé particulier est choisi de manière à ce que son association avec au moins un monomère électrophile dans une composition conduise à la formation d'un film après séchage dans de la composition à température ambiante et à un taux d'humidité relative d'environ 50 %, présentant un module d'Young compris entre 100 et 2000 MPa, mesuré pour une épaisseur de 0,5 mm et avec une vitesse de traction de 20 mm/min. Cette valeur du module d'Young conduit à l'obtention d'un gainage dit rigide des matières kératiniques.

La composition comprenant une telle association permet de maintenir la douceur et la brillance apportées à la chevelure par ladite composition, et ceci sans ré-application, même après plusieurs lavages de la chevelure.

En outre, cette composition, appliquée sur les matières kératiniques permet l'obtention d'un renforcement desdites matières.

L'application d'une composition comprenant une telle association conduit à la formation in situ d'un revêtement non collant et brillant, rémanent, notamment aux lavages.

De plus, lorsqu'elle est appliquée sur les cheveux, la composition confère à la chevelure, volume et maintien, les cheveux restant de manière surprenante parfaitement individualisés.

L'invention a donc pour objet l'utilisation pour le traitement cosmétique des matières kératiniques, d'une composition filmogène comprenant dans un milieu cosmétiquement acceptable, au moins un monomère électrophile et au moins un polymère non siliconé tel que défini ci-dessous.

Un autre objet de la présente invention consiste en un procédé de traitement cosmétique des matières kératiniques mettant en oeuvre ladite composition.

L'invention a encore pour objet un kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un copolymère non siliconé séquencé tel que défini ci-dessous.

D'autres objets, caractéristiques, aspects et avantages de l'invention apparaîtront encore plus clairement à la lecture de la description et de l'exemple qui suit.

Selon l'invention, la composition comprend dans un milieu cosmétiquement acceptable, au moins un monomère électrophile et au moins un polymère non siliconé tel que le film obtenu à partir de la composition par séchage à température ambiante et avec un taux d'humidité relative d'environ 50 % présente un module d'Young compris entre 100 et 2000 MPa, mesuré pour une épaisseur de 0,5 mm et avec une vitesse de traction de 20 mm/min.

Par température ambiante, on entend au sens de la présente invention, une température comprise dans l'intervalle allant de 20 à 24 °C.

Le polymère non siliconé présente une chaîne principale comprenant des atomes de carbone et d'hydrogène, pouvant être interrompue par un ou plusieurs atomes hétéroatomes tels que O, N, P et S, et pouvant comporter une ou plusieurs fonctions en bout de chaîne ou latérale.

Le polymère non siliconé utilisé dans la présente invention peut être dendritique, linéaire, ramifiée, séquencé, en forme de peigne ou d'étoile. Il peut présenter un ou plusieurs types de motifs de répétition, et être un homopolymère ou copolymère statistique, séquencé ou alterné. De préférence, il s'agit d'un copolymère séquencé.

Le polymère comprend au moins cinq motifs de répétition liés par des liaisons covalentes.

Par "non siliconé", on entend au sens de la présente invention un polymère qui ne contient pas d'enchaînement -Si-O-Si-.

Les homopolymères ou copolymères pouvant être utilisés dans la présente invention peuvent se solubiliser ou se disperser spontanément ou par neutralisation dans l'eau. Ils peuvent être constitués d'au moins deux blocs à caractère hydrophile de composition chimique différente, ou bien d'au moins un bloc à caractère hydrophile et d'au moins un bloc à caractère hydrophobe. Ils peuvent être anioniques, cationiques, non ioniques ou amphotères.

Lorsqu'ils se solubilisent ou se dispersent spontanément dans un milieu anhydre, les homopolymères ou copolymères pouvant être utilisés dans la présente invention, peuvent être constitués d'au moins deux blocs à caractère hydrophobe de composition chimique différente, ou bien d'au moins un bloc à caractère hydrophobe et d'au moins un bloc à caractère hydrophile. Ils peuvent être anioniques, cationiques, non ioniques ou amphotères.

Par homopolymère ou copolymère hydrosoluble ou hydrodispersible ou liposoluble, on entend un homopolymère ou copolymère qui, à la concentration de 0,1 % en poids en matière active dans l'eau ou dans un solvant anhydre, à 25 °C, conduit spontanément ou après neutralisation à l'aide d'un acide ou d'une base à une solution ou suspension macroscopiquement homogène, transparente ou translucide, c'est-à-dire présentant une transmittance à une longueur d'onde de 500 nm, à travers un échantillon de 1 cm d'épaisseur

Par bloc à caractère hydrophile, on entend un bloc constitué par au moins 75 % en moles de monomères hydrosolubles et/ou hydrosolubilisables par neutralisation. Le bloc à caractère hydrophile comprend au plus 25 % en moles de monomères non hydrosolubles, de préférence au plus 10 % en moles et mieux encore au plus 5 % en moles.

Par bloc à caractère hydrophobe, on entend un bloc constitué par au moins 75 % en moles de monomères non hydrosolubles. Le bloc à caractère hydrophobe comprend au plus 25 % en moles de monomères hydrosolubles, de préférence au plus 10 % en moles et mieux encore au plus 5 % en moles.

A titre d'exemples de monomère utilisable dans les polymères de l'invention, on peut notamment citer ceux décrits dans la demande FR 2 840 205.

De préférence, les polymères non siliconés utilisés dans l'invention sont des copolymères séquencés linéaires amphiphiles.

Les monomères hydrosolubles formant le ou les blocs hydrophiles des copolymères séquencés pouvant être utilisés dans la présente invention peuvent être de nature anionique, non-ionique ou cationique et peuvent être utilisés seuls ou sous forme de mélange contenant deux ou plusieurs monomères différents.

On peut citer à titre d'exemples de monomères hydrosolubles anioniques, les acides carboxyliques à insaturation éthylénique, tels que l'acide acrylique, l'acide méthacrylique, l'acide itaconique, l'acide fumarique, l'acide crotonique et l'acide maléique, l'acide 2-acrylamido-2-méthylpropanesulfonique, l'acide styrènesulfonique, l'acide vinylsulfonique et l'acide vinylphosphonique.

Les monomères hydrosolubles non-ioniques englobent, entre autres, l'acrylamide, les acrylamides N-alkylés en C₁-C₆ ou N,N-dialkylés en C₁-C₃, l'acrylate de polyéthylèneglycol, le méthacrylate de polyéthylèneglycol, le N-vinylacétamide, le N-méthyl-N-vinlacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène, l'acrylate d'hydroxyéthyle, l'acrylate d'hydroxypropyle, le méthacrylate d'hydroxyéthyle et le méthacrylate de d'hydroxpropyle.

Enfin, les monomères hydrosolubles cationiques englobent par exemple le chlorure de diméthyldiallylammonium, le chlorure de méthylvinylimidazolium, la 2-vinylpyridine, la 4-vinylpyridine, la 2-méthyl-5-vinylpyridine, les halogénures de N-(alkyle en C₁-C₄)-4-vinylpyridinium tels que l'iodure de N-méthyl-4-vinylpyridinium, al vinylamine, les monomères de formule

H₂C=CX₁-CO-X₂

dans laquelle
X₁ représente un atome d'hydrogène ou un groupe méthyle,
X₂ représente un groupe hydrocarboné linéaire ou ramifié en C₁-C₆ portant au moins une fonction amine primaire, secondaire ou tertiaire ou au moins un atome d'azote quaternaire, ou un groupe de formule NHR₂ ou de formule NX₃X₄ où X₃ et X₄ représentent indépendamment l'un de l'autre chacun un groupe hydrocarboné en C₁₋C₆, linéaire ou ramifié, portant au moins une fonction amine primaire, secondaire ou tertiaire ou au moins un atome d'azote quaternaire.

Les monomères insolubles dans l'eau (non hydrosolubles) formant le ou les blocs hydrophobes des copolymères séquencés sont choisis de préférence parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₁-C₁₀, d'aryle en C₆-C₁₀ ou d'aralkyle en C₆-C₁₀ et les méthacrylates d'alkyle en en C₁-C₁₀, d'aryle en C₆-C₁₀ ou d'aralkyle en C₆-C₁₀, comme par exemple les (méth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R" et les allyléthers de formule CH₂=CH-CH₂-O-R", où R" représente un groupe alkyle en C₁-C₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroalkylates d'alkyle.

Le(s) polymère(s) non siliconé(s) tel que décrit(s) ci-dessus, est ou sont présent(s) dans les compositions cosmétiques de l'invention, en une concentration comprise entre 0,05 et 99% en poids, de préférence entre 0,1 et 95 % en poids, et mieux encore entre 0,2 et 30 % en poids, par rapport au poids total de la composition.

Par monomère électrophile, on entend un monomère capable de polymériser par polymérisation anionique en présence d'un agent nucléophile tel que par exemple, les ions hydroxyles (OH-) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
(i) les dérivés benzylidene malononitrile (A), le 2-(4-chlorobenzylidene)-malononitrile (A1), le 2-cyano-3-phényl acrylate d'éthyle (B), le 2-cyano-3-(4-chloro-phényl) acrylate d'éthyle (B1) tels que décrits dans Sayyah, J. Polymer Research, 2000, p97 : 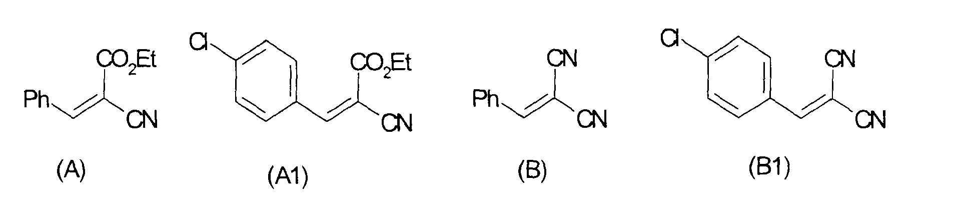
(ii) les dérivés de méthylidenemalonates comme :
   - le 2-méthylene-malonate de diéthyle (C) tel que décrit par Hopff, Makromoleculare Chemie, 1961, p95, par De Keyser, J. Pharm. Sci, 1991, p67 et par Klemarczyk, Polymer, 1998, p173 : 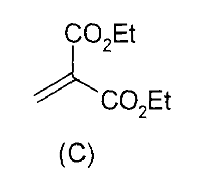
   - le 2-éthoxycarbonylméthyleneoxycarbonyl acrylate d'éthyle (D) tel que décrit par Breton, Biomaterials, 1998, p271 et Couvreur, Pharmaceutical Research, 1994, p1270 : 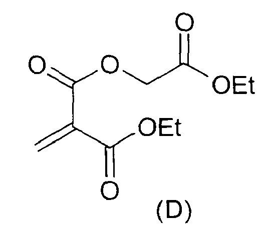
(iii) les dérivés itaconate et itaconimide comme :
   - l'itaconate de diméthyle (E) tel que décrit par Bachrach, European Polymer Journal, 1976, p563 : 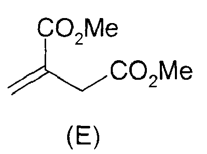
   - le N-butyl itaconimide (F), le N-(4-tolyl) itaconimide (G), le N-(2-ethylphenyl) itaconimide (H), le N-(2,6-diethylphenyl) itaconimide (I) tel que décrits par Wanatabe, J.Polymer Science : Part A :Polymer chemistry, 1994, p2073 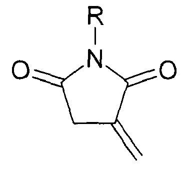 R= Bu (F), 4-tolyl (G), 2-ehylphenyl (H), 2,6-diethyphenyl (I)
(iv) les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O) tel que décrits par Gipstein, J.Org.Chem, 1980, p1486 et les dérivés 1,1-bis-(methylsulfonyl)ethylene (P), 1-acetyl-1-methyl sulfonyl ethylene (Q) , α-(methylsulfonyl) vinyl sulfonate de methyle (R) , α-methylsulfonylacrylonitrile (S) tel que décrits par Shearer, US patent US2748050 : 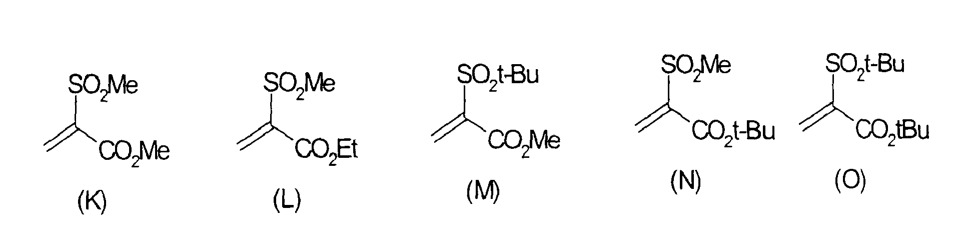 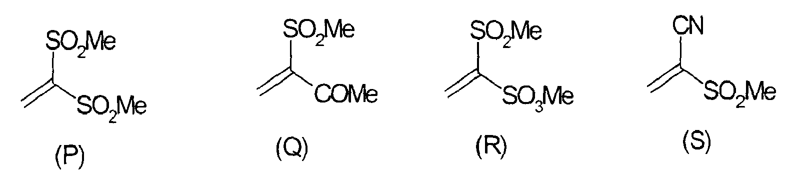
(v) les dérivés méthyl vinyl sulfone (T) et phényl vinyl sulfone (U) tel que décrits par Boor , J.Polymer Science, 1971, p249 : 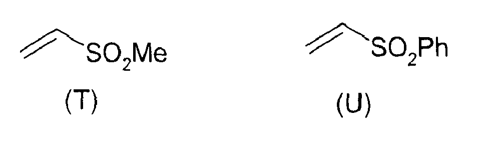
(vi) le dérivé phényl vinyl sulfoxide (V) tel que décrit par Kanga, Polymer preprints (ACS, Divison of Polymer Chemistry), 1987, p322 : 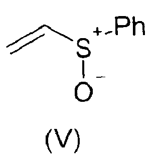
(vii) le dérivé 3-methyl-N-(phenylsulfonyl)-1-aza-1,3-butadiene (W) tel que décrit par Bonner, Polymer Bulletin, 1992, p517 : 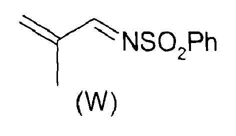
(viii) les dérivés acrylates et acrylamides comme :
   - le N-propyl-N-(3-triisopropoxysilylpropyl)acrylamide (X) et N-propyl-N-(3-triethoxysilylpropyl)acrylamide (Y) tel que décrit par Kobayashi, Journal of Polymer Science, Part A: Polymer Chemistry, 2005, p2754 : 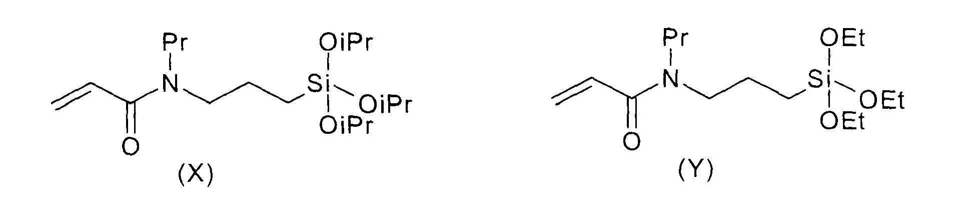
   - le 2-hydroxyethyl acrylate (Z) et le 2-hydroxyethyl méthacrylate (AA) tel que décrits par Rozenberg, International Journal of Plastics Technology, 2003, p17 : 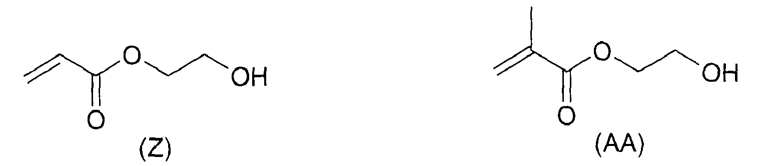
   - le N-butyl acrylate (AB) tel que décrit par Schmitt, Macromolecules, 2001, p2115, et le tert-butyl acrylate (AC) tel que décrit par Ishizone, Macromolecules, 1999, p955 :

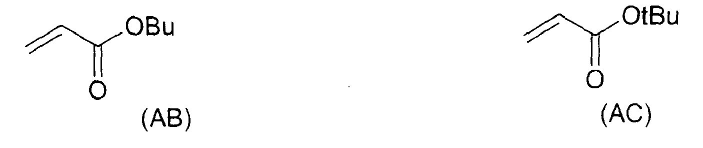

Le monomère électrophile, ou électro-attracteur, utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères électrophiles présentant au moins deux groupes électro-attracteurs, on peut citer les monomères de formule (I) : 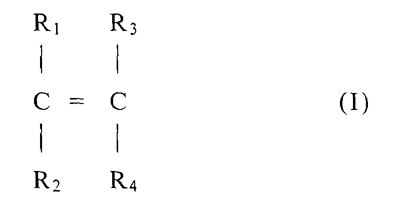 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, - OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène et un résidu de polymère pouvant être obtenu par polymérisation radicalaire ou par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)ₙ-(CF₂)ₘ-CF₃ ou - (CH₂)ₙ-(CF₂)ₘ-CHF₂ avec n=1 à 20 et m= 1 à 20.

Les substituants R₁ à R₄ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (II) : 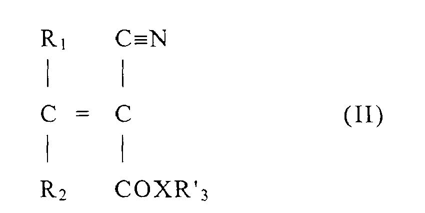 X désignant NH,S,O,
R₁ et R₂ ayant les mêmes significations que précédemment,
R'₃ pouvant désigner un atome d'hydrogène ou un groupe R tel que défini pour la formule (1).

De préférence, X désigne O.

A titre de composés de formule (II), on peut citer les monomères :
a) appartenant à la famille des 2-cyanoacrylates de polyfluoroalkyle en C₁₋₂₀ tels que :
   l'ester 2,2,3,3-tétrafluoropropylique de l'acide 2-cyano-2-propénoïque de formule : 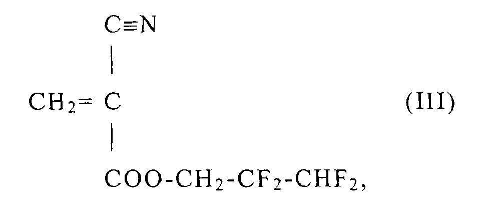
   ou encore l'ester 2,2,2-trifluoroéthylique de l'acide 2-cyano-2-propénoïque de formule : 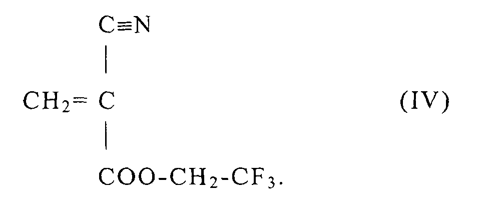
b) les cyanoacrylates d'alkyle en C₁-C₁₀ ou d'(alcoxy en C₁₋C₄)(alkyle en C₁-C₁₀).
   On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères le plus particulièrement préférés sont ceux de formule (V) et leurs mélanges : 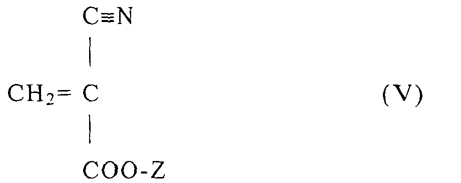 dans laquelle : Z=-(CH₂)₇-CH₃,
-CH(CH₃)-(CH₂)₅-CH₃,
-CH₂-CH(C₂H₅)-(CH₂)₃₋CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

Par milieu cosmétiquement acceptable, on entend un milieu compatible avec les matières kératiniques telles que les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C_{10;} l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique et l'alcool béhénylique, les acides gras en C₁₀-C₃₀ tels que l'acide laurique et l'acide stéarique, les amides gras en C₁₀-C₃₀ tels que le diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 10⁵ Pa (760mm de Hg).

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, tel que, par exemple, des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des gaz propulseurs et des épaississants minéraux ou organiques tels que le benzylidène-sorbitol et les N-acylaminoacides.

Ces agents peuvent être éventuellement encapsulés. La capsule peut être de type polycyanoacrylate.

La composition est utilisée de préférence en présence d'un agent nucléophile, pour le traitement cosmétique des matières kératiniques, et plus particulièrement des cheveux.

Le procédé de traitement cosmétique selon l'invention comprend l'application d'une composition telle que définie ci-dessus sur les matières kératiniques, en présence d'un agent nucléophile tel que défini ci-dessous.

Les agents nucléophiles susceptibles d'initier la polymérisation anionique sont des systèmes connus en eux-mêmes, capables de générer un carbanion au contact d'un monomère électrophile. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

Les agents nucléophiles particulièrement préférés sont les ions hydroxyle, notamment ceux présents dans l'eau. Cette eau peut être apportée par une humidification préalable des matières kératiniques.

Selon un mode préféré de réalisation, la composition comprenant le monomère électrophile est exempte d'agent nucléophile.

Selon un autre mode préféré de réalisation, l'agent nucléophile est apporté par une seconde composition, appliquée sur ou sous le dépôt formé par l'application de la composition comprenant le monomère électrophile.

Il est également possible, afin de moduler la cinétique de réaction, de préalablement humidifier les matières kératiniques, et notamment les fibres kératiniques, à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les matières kératiniques, et notamment les fibres kératiniques, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des matières kératiniques, et notamment des fibres kératiniques, par transformation chimique de la matière kératinique.

A titre d'exemple de transformation chimique, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6 224 622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions telles que décrites ci-dessus peut être suivie ou non d'un rinçage.

Elles peuvent se présenter sous la forme de lotion, de spray, de mousse, et être appliquées comme shampoing ou après-shampoing.

Un autre objet de l'invention est une composition comprenant, dans un milieu cosmétiquement acceptable, au moins un monomère électrophile et au moins un copolymère non siliconé séquencé tel que décrit ci-dessus.

Par copolymère séquencé, on entend au sens de la présente invention, tout copolymère comportant au moins deux blocs distincts. De préférence, les polymères séquencés de l'invention sont linéaires. De tels polymères sont décrits ci-dessus.

L'invention a encore pour objet un kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un copolymère non siliconé séquencé tel que décrit ci-dessus.

Un autre objet de la présente invention est un procédé de traitement cosmétique des matières kératiniques, comprenant au moins deux étapes, une étape comprenant l'application du polymère non siliconé tel que défini ci-dessus, et une autre étape comprenant l'application d'au moins un monomère électrophile tel que défini ci-dessus, l'ordre des étapes étant indifférent.

Un mode de réalisation particulier de l'invention consiste en ce que l'application du polymère non siliconé se fait avant l'application d'au moins un monomère électrophile.

L'exemple suivant est donné à titre illustratif de la présente invention.

Dans l'exemple suivant, toutes les quantités sont indiquées en pour cent en poids de matière active par rapport au poids total de la composition, sauf indication contraire.

### EXEMPLES

### Exemple 1 :

On prépare la composition suivante, conforme à l'invention :
- Cynaoacrylate de 2-octyle (1) 49 %
- Poly(méthacrylate de méthyle) (2) 50 %
- Monoéthanolamine 1 %
(1) commercialisé par la société Chemence
(2) billes de polyméthacrylate de méthyle, ACROS Chemical, référence 178765000

On dépose la composition sur une matrice de téflon. On fait sécher le film à température ambiante et sous une humidité relative de 50 %. L'épaisseur du film obtenu est de 0,5 mm.

On mesure ensuite le module de Young du film obtenu avec un appareil LLOYD modèle RLSK, pour une vitesse de traction de 20 mm/min. La valeur est d'environ 200 MPa.

La composition appliquée sur les cheveux et séchée confère un gainage rigide résistant aux shampoings.

### Exemple 2 :

Cet exemple met en évidence le caractère rigide du gainage obtenu au moyen des compositions selon l'invention.

On utilise les compositions suivantes, conformes à l'invention:

### Composition A: monomère méthylheptylcyanoacrylate et milieu huileux siliconé et polymère PVP

### Composition A1 :

- Polyvinylpyrrolidone LUVISKOL de BASF 5 %
- Eau Qsp 100

### Composition A2 :

- Méthylheptylcyanoacrylate (1) 10 %
- DC245 Fluid de Dow Corning 45 %
- DC 1501 Fluid de Dow Corning 45 %
(1) commercialisé par la société Chemence

La composition A1 est appliquée sur des cheveux propres et humides. Puis on applique la composition A2. Les cheveux sont ensuite séchés pendant 30 minutes au casque.

### Composition B: monomère methylheptylcyanoacrylate et milieu huileux siliconé et polymère PS

### Composition B1 :

- Dispersion de polystyrene MODAREZ OS-197 5 % de SYNTHRON
- Eau Qsp 100

### Composition B2 :

- Méthylheptylcyanoacrylate (1) 10 %
- DC245 Fluid de Dow Corning 45 %
- DC 1501 Fluid de Dow Corning 45 %
(1) commercialisé par la société Chemence

La composition B1 est appliquée sur les cheveux propres et humides. Puis on applique la composition B2. Les cheveux sont ensuite séchés pendant 30 minutes au casque.

### Composition C: monomère méthylheptylcyanoacrylate et milieu huileux siliconé et polymère PMMA

### Composition C1 :

- Polyméthylmethacrylate de ACROS , 5 % référence 178765000
- Méthyl éthyl cétone Qsp 100

### Composition C2 :

- Méthylheptylcyanoacrylate (1) 10 %
- DC245 Fluid de Dow Corning 45 %
- DC 1501 Fluid de Dow Corning 45 %
(1) commercialisé par la société Chemence

La composition C1 est appliquée sur des cheveux propres et humides. Puis on applique la composition C2. Les cheveux sont ensuite séchés pendant 30 minutes au casque.

La mesure du module d'Young du gainage formé directement sur les cheveux après application des compositions A, B et C ci-avant est effectuée au moyen de la nanoindentation par microscopie à force atomique (AFM).

Les résultats obtenus sont les suivants :

| | Module d'Young |
|---|---|
| Composition A | 428MPa (+/- 227) |
| Composition B | 360MPa (+/- 119) |
| Composition C | 236 MPa(+/- 105) |

Les valeurs de modules d'Young obtenues sont toutes supérieures à 100 MPa, ce qui correspond bien à des gainages rigides.

### Exemples 3 à 9 :

Les exemples 3 à 9 ci-après illustrent d'autres compositions conformes à l'invention.

### Exemple 3 :

- Méthylheptylcyanoacrylate (1) 10 %
- DC245 Fluid de Dow Corning 25%
- DC 1501 Fluid de Dow Corning 25%
- Poly(méthacrylate de méthyle)) 39 %
- Monoéthanolamine 1 %
(1) commercialisé par la société Chemence

### Exemple 4 :

- Méthylheptylcyanoacrylate (1) 10 %
- Pure olive oil de Arista industries 50%
- Poly(méthacrylate de méthyle) 39 %
- Monoéthanolamine 1 %
(1) commercialisé par la société Chemence

### Exemple 5 :

- Ethoxyethylcyanoacrylate (1) 49 %
- Poly(méthacrylate de méthyle) 50 %
- Monoéthanolamine 1 %
(1) EO 460 commercialisé par la société Tong Shen

### Exemple 6 :

- Butylcyanoacrylate (1) 49 %
- Poly(méthacrylate de méthyle) 50 %
- Monoéthanolamine 1 %
(1) B-60 commercialisé par la société Tong Shen

### Exemple 7 :

- Ethylhexylcyanoacrylate (1) 49 %
- Poly(méthacrylate de méthyle) 50 %
- Monoéthanolamine 1 %
(1) O-60 commercialisé par la société Tong Shen

### Exemple 8 :

- Méthylheptylcyanoacrylate (1) 44.1 %
- Ethylhexylcyanoacrylate (2) 4.9 %
- Poly(méthacrylate de méthyle) 50 %
- Monoéthanolamine 1 %
(1) commercialisé par la société Chemence
(2) O-60 commercialisé par la société Tong Shen

### Exemple 9 :

- Méthylheptylcyanoacrylate (1) 34.3 %
- Butylcyanoacrylate (2) 14.7 %
- Poly(méthacrylate de méthyle) 50 %
- Monoéthanolamine 1 %
(1) commercialisé par la société Chemence
(2) B-60 commercialisé par la société Tong Shen

## Revendications

1. Utilisation pour le traitement cosmétique des matières kératiniques, d'une composition filmogène comprenant dans un milieu cosmétiquement acceptable, au moins un monomère électrophile et au moins un polymère non siliconé tel que le film obtenu à partir de la composition par séchage à température ambiante et avec un taux d'humidité relative d'environ 50 % présente un module d'Young compris entre 100 et 2000 MPa, mesuré pour une épaisseur de 0,5 mm et avec une vitesse de traction de 20 mm/min.

2. Utilisation selon la revendication 1, **caractérisée en ce que** le polymère non siliconé est choisi parmi les homopolymères et les copolymères statistiques, séquencés ou alternés, et de préférence parmi les copolymères séquencés.

3. Utilisation selon la revendication 2, **caractérisée en ce que** le polymère non siliconé est choisi parmi les copolymères amphiphiles linéaires séquencés.

4. Utilisation selon la revendication 3, **caractérisée en ce que** le copolymère amphiphile comprend des monomères hydrosolubles de nature anionique, non-ionique ou cationique.

5. Utilisation selon la revendication 3 ou 4, **caractérisée en ce que** le copolymère amphiphile comprend des monomères non hydrosolubles choisis parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₁-C₁₀, d'aryle en C₆-C₁₀ ou d'aralkyle en C₆-C₁₀ et les méthacrylates d'alkyle en en C₁-C₁₀, d'aryle en C₆-C₁₀ ou d'aralkyle en C₆-C₁₀, comme par exemple les (méth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R" et les allyléthers de formule CH₂=CH-CH₂-O-R", où R" représente un groupe alkyle en C₁-C₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroalkylates d'alkyle.

6. Utilisation selon l'une quelconque des revendications précédentes, **caractérisée en ce que** le polymère non siliconé est contenu en une quantité comprise entre 0,05% et 99% en poids, de préférence entre 0,1 et 95 % en poids par rapport au poids total de la composition.

7. Utilisation selon l'une quelconque des revendications précédentes, **caractérisée en ce que** le ou les monomères électrophiles répondent à la formule : 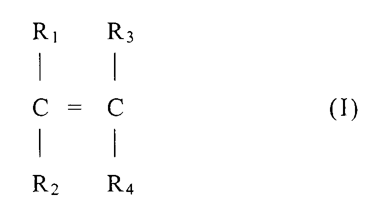 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR, -COOR, -COR, - SH, -SR, -OH et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, -S(R)₂⁺, - SH₂⁺, -NH₃⁺, -NO₂, -SO₂R, -C≡N, -COOH, -COOR, -COSR, -CONH₂, - CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle et aryloxy.
R désigne un groupe hydrocarboné saturé ou non, linéaire, ramifié ou cyclique, comportant de 1 à 20 atomes de carbone et contenant éventuellement un ou plusieurs atomes d'azote, d'oxygène, de soufre, et éventuellement substitué par un ou plusieurs groupements choisi parmi -OR', -COOR', -COR', -SH, -SR', -OH, les atomes d'halogène, et un résidu de polymère pouvant être obtenu par polymérisation radicalaire ou par polycondensation ou par ouverture de cycle, R' désignant un groupe alkyle en C₁-C₁₀.

8. Utilisation selon la revendication 7, **caractérisée en ce que** le ou les monomères électrophiles sont choisis parmi les composés de formule : 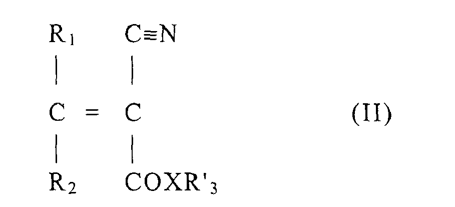 X désignant NH, S, O,
R₁ et R₂ sont tels que définis dans la revendication 7,
R'₃ pouvant désigner un atome d'hydrogène ou un groupe R tel que défini dans la revendication 7.

9. Utilisation selon la revendication 8, **caractérisée par le fait que** le ou les monomères sont choisis parmi les 2-cyanoacrylates de polyfluoroalkyle en C₁₋₂₀, les cyanoacrylates d'alkyle en (C₁-C₁₀) ou d'(alcoxy en C₁-C₄)(alkyle en C₁-C₁₀).

10. Utilisation selon la revendication 9, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

11. Utilisation selon la revendication 10, **caractérisée en ce que** le ou les monomères électrophiles répondent à la formule (V) : 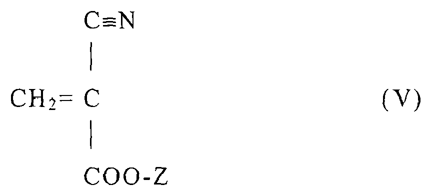 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

12. Utilisation selon l'une quelconque des revendications précédentes, **caractérisée en ce que** le ou les monomères électrophiles sont fixés de façon covalente sur des supports tels que des polymères, des oligomères ou des dendrimères.

13. Utilisation selon l'une quelconque des revendications précédentes, **caractérisée en ce que** la quantité de monomère électrophile va de 0,001 à 80 % en poids, de préférence de 0,1 à 40 % en poids par rapport au poids total de la composition.

14. Utilisation selon l'une quelconque des revendications précédentes, **caractérisée en ce que** le milieu cosmétiquement acceptable est anhydre.

15. Utilisation selon la revendication 14, **caractérisée en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀. les esters d'alcool gras en C₁₀-C₃₀ et leurs mélanges.

16. Utilisation selon l'une quelconque des revendications précédentes, **caractérisée en ce que** la composition contient des inhibiteurs de polymérisation.

17. Utilisation selon la revendication 16, **caractérisée en ce que** les inhibiteurs sont des inhibiteurs de polymérisation anioniques et/ou radicalaires.

18. Utilisation selon la revendication 16 ou 17, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges.

19. Utilisation selon l'une quelconque des revendications 16 à 18, **caractérisée en ce que** les inhibiteurs de polymérisation sont présents en des quantités allant de 10 ppm à 20 % par rapport au poids total de la composition.

20. Utilisation selon l'une quelconque des revendications précédentes, en présence d'un agent nucléophile.

21. Utilisation selon la revendication 20, **caractérisée en ce que** l'agent nucléophile est choisi parmi les composés moléculaires, les oligomères, les dendrimères ou polymères possédant des fonctions nucléophiles choisies parmi : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

22. Utilisation selon la revendication 20 ou 21, **caractérisée en ce que** l'agent nucléophile est constitué d'ions hydroxyle, notamment ceux présents dans l'eau .

23. Utilisation selon l'une quelconque des revendications précédentes, **caractérisée en ce que** la composition contient en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, non ioniques ou amphotères, les polymères fixants ou non, les polyols, les protéines, des vitamines, les colorants directs ou d'oxydation, les agents nacrants, les gaz propulseurs et les agents épaississants minéraux ou organiques.

24. Utilisation selon la revendication 23, **caractérisée en ce que** l'agent est encapsulé.

25. Utilisation de la composition selon l'une quelconque des revendications précédentes, pour le traitement cosmétique des fibres kératiniques, et de préférence des cheveux.

26. Composition comprenant dans un milieu cosmétiquement acceptable, au moins un monomère électrophile, et au moins un copolymère non siliconé séquencé tel que le film obtenu à partir de la composition par séchage à température ambiante et avec un taux d'humidité relative d'environ 50 % présente un module d'Young compris entre 100 et 2000 MPa, mesuré pour une épaisseur de 0,5 mm et avec une vitesse de traction de 20 mm/min.

27. Composition selon la revendication 26, **caractérisée en ce que** le copolymère non siliconé séquencé comprend des monomères hydrosolubles de nature anionique, non-ionique ou cationique.

28. Composition selon la revendication 26 ou 27, **caractérisée en ce que** le copolymère non siliconé séquencé comprend des monomères non hydrosolubles choisis parmi les monomères vinylaromatiques tels que le styrène et ses dérivés alkylés comme le 4-butylstyrène, l'α-méthylstyrène et le vinyltoluène, les diènes tels que le butadiène et le 1,3-hexadiène, et les dérivés alkylés des diènes tels que l'isoprène et le diméthylbutadiène, le chloroprène, les acrylates d'alkyle en C₁-C₁₀, d'aryle en C₆-C₁₀ ou d'aralkyle en C₆-C₁₀ et les méthacrylates d'alkyle en en C₁-C₁₀, d'aryle en C₆-C₁₀ ou d'aralkyle en C₆-C₁₀, comme par exemple les (méth)acrylates de méthyle, d'éthyle, de n-butyle, de 2-éthylhexyle, de tert-butyle, d'isobornyle, de phényle ou de benzyle, l'acétate de vinyle, les vinyléthers de formule CH₂=CH-O-R" et les allyléthers de formule CH₂=CH-CH₂-O-R", où R" représente un groupe alkyle en C₁-C₆, l'acrylonitrile, le chlorure de vinyle, le chlorure de vinylidène, la caprolactone, l'éthylène, le propylène, les monomères vinyliques fluorés ou à chaîne perfluorée, tels que les acrylates et méthacrylates de fluoroalkyle ou les α-fluoroalkylates d'alkyle.

29. Procédé de traitement cosmétique des matières kératiniques comprenant l'application d'une composition telle que définie dans l'une quelconque des revendications précédentes 1 à 24, sur les matières kératiniques, en présence d'un agent nucléophile.

30. Procédé selon la revendication 29, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NN₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle, Ar représentant un groupe aryle et R représentant un groupe alkyle en C₁-C₁₀.

31. Procédé selon la revendication 30, **caractérisé en ce que** l'agent nucléophile est constitué d'ions hydroxyle, notamment ceux présents dans l'eau.

32. Procédé selon l'une quelconque des revendications 29 à 31, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

33. Procédé selon l'une quelconque des revendications 29 à 31, **caractérisé en ce que** les matières kératiniques sont pré-imprégnées à l'aide d'un agent nucléophile différent de l'eau.

34. Procédé selon l'une quelconque des revendications 29 à 31, **caractérisé en ce que** les fibres kératiniques sont préalablement réduites avant application de la composition.

35. Procédé selon l'une des revendications 29 à 34, **caractérisé en ce que** l'application de la composition est suivie d'un rinçage.

36. Procédé selon l'une des revendications 29 à 35, **caractérisé en ce que** les matières kératiniques sont les cheveux.

37. Kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un copolymère non siliconé séquencé tel que défini dans l'une quelconque des revendications 1 à 5.

38. Procédé de traitement cosmétique des matières kératiniques, comprenant au moins deux étapes, une étape comprenant l'application d'un polymère non siliconé tel que défini dans l'une quelconque des revendications 1 à 5, et une autre étape comprenant l'application d'au moins un monomère électrophile tel que défini dans l'une quelconque des revendications 7 à 12.

39. Procédé selon la revendication 38, **caractérisé en ce que** l'application du polymère non siliconé se fait avant l'application d'au moins un monomère électrophile.
